(19) Europäisches Patentamt

European Patent Office

Office européen des brevets

(11) EP 0 588 857 B1

(12) **FASCICULE DE BREVET EUROPEEN**

(45) Date de publication et mention
de la délivrance du brevet:
**15.01.1997 Bulletin 1997/03**

(21) Numéro de dépôt: 92911630.9

(22) Date de dépôt: 03.06.1992

(51) Int. Cl.$^6$: **A01N 65/00**, A01N 43/90
// (A01N65/00, 25:04, 25:10,
25:12, 25:16), (A01N43/90,
25:04, 25:10, 25:12, 25:16)

(86) Numéro de dépôt international:
**PCT/FR92/00490**

(87) Numéro de publication internationale:
**WO 92/21242 (10.12.1992 Gazette 1992/31)**

(54) **COMPOSITION INSECTICIDE**

**INSEKTIZIDE MITTEL**

**INSECTICIDE COMPOSITION**

(84) Etats contractants désignés:
**BE CH DE ES GR IT LI MC**

(30) Priorité: **05.06.1991 FR 9106797**

(43) Date de publication de la demande:
**30.03.1994 Bulletin 1994/13**

(73) Titulaires:
• **Gueyne, Jean**
**MC-98000 Monte-Carlo (MC)**
• **Seguin, Marie-Christine**
**MC-98000 Monte-Carlo (MC)**

(72) Inventeurs:
• **Gueyne, Jean**
**MC-98000 Monte-Carlo (MC)**

• **Seguin, Marie-Christine**
**MC-98000 Monte-Carlo (MC)**

(74) Mandataire: **Bonneau, Gérard**
**Cabinet Bonneau,**
**Conseil en Propriété Industrielle,**
**7, Avenue Gazan**
**06600 Antibes (FR)**

(56) Documents cités:
EP-A- 0 274 961          EP-A- 0 409 690
FR-A- 1 572 106          FR-A- 2 304 326
FR-A- 2 448 856          FR-A- 2 526 632
GB-A-  559 647           GB-A-  999 960
US-A- 1 945 312          US-A- 1 967 024
US-A- 2 194 446          US-A- 3 539 465
US-A- 3 549 555

## Description

L'invention concerne une nouvelle famille de pesticides ou insecticides et un moyen pour la destruction d'insectes, notamment des parasites du corps humain ou animal; elle vise plus particulièrement - des produits sarcopticides, humains et animaux et, plus particulièrement, destinés à la gale du mouton - des produits pédiculicides et, en premier lieu, des compositions pour la destruction des poux et de leurs lentes. Elle permet de lutter contre les acariens, Dermatophagoides pteronyssinus ou/et farinae, le varon ou Hypoderma bovis et tincatum, et contre le ver ou larves Cochlionyiae hominivorax.

Il existe un certain nombre de produits connus dans ce domaine, par exemple des organochlorés tels que Lindane, D.D.T., des organophosphorés, tels que Malathion, Parathion et les pyréthrines naturelles ou de synthèse, associées ou non à du pipéronyl butoxyde. Tous ces insecticides sont plus ou moins efficaces, car les insectes acquièrent une résistance par accoutumance. De plus, à part la pyréthrine, ils laissent des résidus et ne sont pas biodégradables. Les organochlorés sont indestructibles et se retrouvent dans les eaux et dans la nature, sont retenus dans les graisses animales et détruisent la couche de myéline des nerfs. Avec les organophosphorés, on observe un phénomène de sélection de souches résistantes, nécessitant l'emploi d'organophosphorés plus performants et par conséquent plus toxiques.

En fait malgré tous ces produits, on assiste à l'heure actuelle à des véritables invasions d'octoparasites dans différents pays; la raison en est que, les oeufs, notamment ceux des pédiculides, étant très difficiles à détruire, et les insectes eux-mêmes devenant à la longue résistants aux substances actives employées, une période d'accalmie, après la mort de la majeure partie des insectes, est généralement suivie de l'éclosion d'une nouvelle génération de parasites. Cela d'autant plus que, sous peine de l'intoxication à court et à long terme, du sujet traité, il n'est guère possible de prolonger l'action de l'insecticide. On notera que, dans la technique pratiquée en ce domaine, on cherche à effectuer le traitement rapidement, de façon à laisser le produit actif le moins longtemps possible au contact du corps de la personne ou de l'animal traité; ainsi considère-t-on comme appropriés des produits qui détruisent après une application la totalité des parasites.

Jusqu'à présent, des extraits de Derris Elliptica, Téphrosia Vogelii et Lonchocarpus Nicou, titrant environ 40 % de roténone ont été utilisés pour le traitement des pédiculoses. Cet extrait est une oléorésine très irritante pour la peau et toxique per os, fragile, puisque le principe actif, la roténone, est détruite par la lumière et difficile d'emploi car très peu soluble dans les solvants compatibles avec une application cutanée.

Malgré cela, la présente invention permet d'obtenir, à partir de la roténone, une composition qui convient parfaitement à des applications cutanées, sans aucun risque d'irritation ou d'intoxication. L'invention réalise, en effet, une composition non seulement inoffensive vis-à-vis de la peau humaine ou animale, mais, de plus biodégradable et - par conséquent - ne laissant subsister aucun résidu sur les sujets traités. Un exemple particulier, important, de cet avantage se trouve dans le traitement de la gale du mouton : la laine des animaux traités ne retient pas la roténoide d'une composition suivant l'invention, alors qu'un insecticide tel que "Lindane" se retrouve jusque dans les vêtements fabriqués avec la laine des moutons qui furent traités contre la gale.

L'invention résulte de la constatation imprévue que les inconvénients de l'insecticide à base de roténone disparaissent, lorsque ce produit ne contient plus ou contient tout au plus, une proportion très fortement diminuée des dérivés ou homologues qui, normalement, accompagnent les extraits rotenoniques des plantes.

Ainsi faut-il éliminer, ou réduire à une teneur minimum, les divers composés, connus sous des noms comme sumatrol, biliptone, degueline toxicarol, malaccol et similaires, que l'on trouve habituellement à côté de la roténone dans les extraits naturels. Par contre, non seulement il n'y a pas lieu d'éliminer la dihydroroténone $C_{23}H_{24}O_6$ porteuse de 2H de plus que la roténone $C_{23}H_{22}O_6$, mais - selon un trait particulier de l'invention - on a intérêt à l'employer conjointement avec la roténone, ou seule, dans les compositions insecticides visées.

En admettant pour la roténone la structure de tétrahydro-1,2,12,12a diméthoxy-8,9 [méthyl-1-éthényl]-2 benzo[1]-pyrano furol[3,4,b] benzoyran[2,3,h][1] one 6 (6aH), celle de la dihydroroténone correspondante serait similaire, mais avec une fonction alcool en 6 [6aH] au lieu de cétone.

Pour assurer les avantages des compositions suivant l'invention, la roténone ou/et la dihydroroténone utilisée ne doit pas contenir plus de 10 % en poids de dérivés cétoniques, tels que sumatrol, biliptone, degueline et autres, cités plus haut. De préférence, le produit suivant l'invention titre au moins 99 % de roténone pure ou/et de dérivé dihydro de celle-ci.

Les insecticides suivant l'invention étant employés en solution dans des solvants organiques, ceux-ci jouent un rôle important : ils doivent être inoffensifs pour les humains et pour les animaux autres qu'insectes, ce qui élimine les solvants chlorés; ils doivent dissoudre les produits actifs suffisamment pour en donner les concentrations nécessaires, soit en général environ 0,05 à 10 % en poids, et le plus souvent 0,1 à 5 % dans la composition à appliquer comme insecticide.

Par la publication de brevet français 2 448 856, on connaît divers esters des acides phtalique, benzoique, benzylique, phosphoreux, phosphorique et mellitique, qui conviennent bien comme solvants de la roténone et peuvent - par conséquent - être utilisés suivant la présente invention.

Une autre classe de solvants inoffensifs, qui conviennent, comprend des alkylène-glycols, en particulier en $C_2$ à $C_{18}$. Ainsi peut-on employer avantageusement, par exemple, des glycols de propylène, butylène, pentylène, hexylène, octylène, nonylène, décylène etc.

Il existe, en outre, à l'heure actuelle, un certain choix de solvants utiles notamment pour les produits cosmétiques, constitués par des polyalkyl siloxanes linéaires et cycliques; en particulier de tels liquides avec des alkyles en $C_1$ à $C_6$ conviennent à la préparation des compositions insecticides suivant l'invention. On trouve aisément dans le commerce les solvants comme polydiméthyl cyclosiloxanes et l'hexaméthyl disiloxane.

Il est à noter que le dihydro dérivé, ou la roténone réduite, est en général plus soluble dans différents solvants que la roténone elle-même, on peut donc obtenir des solutions plus concentrées, lorsqu'on a besoin d'une action insecticide plus forte.

Selon une variante de l'invention, la composition rotenonique est additionnée d'une faible proportion de phénol, de terpène, ou des deux sortes de tels composés à la fois. Cette addition améliore la pénétration de la roténone dans les recoins où se logent les insectes et dans les oeufs de ceux-ci.

En tant que phénols on peut utiliser, par exemple, les thymol, eugénol, iso-eugénol, gaiacol, résorcinol, hexyl-6 résorcinol et autres.

Parmi les terpènes, convenant comme additifs des compositions suivant l'invention, on peut citer, à titre d'exemple, les myrcène, linalol, menthanes, menthadiènes (terpinènes, terpinolène, limonène), pinènes, camphane, camphène, bornylène, etc. Ils peuvent - bien entendu - être pris sous la forme d'essences qui les contiennent, telles que l'essence de térébenthine, de citronnelle, de lavande ou similaire.

La teneur de la composition en phénols ou/et terpènes n'est pas critique : elle peut varier par exemple entre environ 0,05 et 5 % en poids pour chacun de ces adjuvants. Elle est, le plus souvent, du même ordre que celle de roténone ou/et dihydroroténone.

Une forme d'exécution spéciale de l'invention consiste à inclure dans la composition des sphérules de petites dimensions d'un polymère capable d'adsorber, ou de fixer par d'autres forces de liaison, la roténone ou/et la roténone réduite, à partir de la solution décrite plus haut.

Bien que l'emploi de suspensions de micro- ou nanosphères de polymères, dans des produits cosmétiques, soit connu, son application à des insecticides formés par une solution organique de roténone ou/et dihydroroténone est inattendue. En effet, rien ne pouvait laisser prévoir a priori qu'une telle application apporterait un avantage : or l'expérience a montré qu'en présence des sphérules de polymère dans la composition rotenonique, toute éclosion d'une nouvelle génération d'insectes, quelques jours après le traitement, est évitée. Donc, contrairement à ce qui se passe avec les insecticides connus, les lentes, restant après la destruction des insectes, ne peuvent pas éclore. C'est un résultat surprenant, qui fait des compositions suivant l'invention un moyen remarquable de destruction de parasites sur le corps humain ou animal ; ainsi détruit-on aisément la gale du mouton, les pédiculidés, l'hypoderma bovis, l'hypoderma tincatum, la larve de cochlionyiac hominivorax, de lucilia sericata, de lucilia euprina etc.

Les microsphères de différents polymères, et la technique de leur préparation sont connues. Il n'y a donc pas lieu de les décrire ici. On peut trouver la description de tels produits et de leur mode de préparation, par exemple, dans les brevets FR 1 572 106 et 2 304 326, EP 0 064 967 ou EP 0 274 961; mais il n'y est pas question de réaliser un effet insecticide complet (insectes et leurs oeufs) comme dans la présente invention. Bien que l'invention puisse porter sur des sphérules microscopiques de diverses grosseurs, de préférence les dimensions ne dépassent pas 1000 nm, les tailles préférées se rangent entre 50 et 500 nm ou, mieux encore, entre 60 et 300 nm. L'optimum de la grosseur des particules dépend d'ailleurs de la nature du polymère qui les constitue.

Les particules, convenant à la réalisation de l'invention, peuvent être choisies parmi les diverses microsphères en polymères connus, pleines ou creuses, à condition d'être insolubles dans le liquide véhicule utilisé. Celui-ci est d'ailleurs, le plus souvent, choisi parmi les solvants de la roténone, indiqués plus haut; il peut être tel que, par exemple, un polyol comme glycol ou glycérine, un éther ou ester de polyol, alcool etc.

Ainsi, convient à l'application suivant l'invention des dispersions de microparticules notamment nanosphères, en des polymères comme polysaccharides, polyamides, polyalkylènes, polyarylalkylènes, polyalkylidènes, polysilicones et autres; dans chacune de ces classes nombreux dérivés et copolymères peuvent être employés. Par exemple, on peut utiliser des polysaccharides tels que xanthane, scléroglucane, pectines, amidons, celluloses, cyclodextrines et leurs dérivés comme amylose, amylopectine, carboxyméthyl-cellulose, hydroxy-cellulose, alkyl-celluloses, dextrine, polysiloxanes etc.

En tant que polyaryl-alkylènes, on peut citer le polystyrène et surtout ses copolymères, en particulier avec des esters éthyléniques, notamment acrylates ou méthacrylates de différents alkyles, d'hydroxy-alkyles, avec du méthacryl- ou acrylamide. De même, conviennent les résines vinyliques, par exemple l'acétate de polyvinyle. D'autre part, des polysiloxanes se révèlent fort utiles.

De façon générale, les produits suivant l'invention contiennent en poids 0,1 à 10 %, et plus souvent 0,3 à 3 %, des sphérules définies plus haut, la teneur choisie dépendant de la nature du ou des polymères, de la taille des particules, de leur affinité vis-à-vis des substances actives et des nature et proportion du liquide de la dispersion.

D'après ce qui précède, la composition suivant l'invention, est constituée, pondéralement, en principe, de : 0,05 à 10 % de roténone pure ou/et de roténone pure, réduite, de 0,1 à 10 % de sphérules en polymère, et de 80 à 99,4 % de liquide ou mélange de liquides tenant en solution le composé roténonique et en suspension lesdites sphérules.

Un mode opératoire pratique, pour la préparation d'une telle composition, comprend les étapes suivantes : (A) dissolution du composé roténonique dans son solvant spécifique, notamment choisi parmi ceux qui figurent plus haut, dans la présente description; (B) dispersion de sphérules dans un non solvant; mélange de la dissolution (A) avec la suspension (B); souvent il y a lieu, en outre, d'ajouter un solvant (C) afin d'éviter la précipitation du composé roténonique du fait de l'addition du non solvant (B).

Ainsi, à titre d'exemple non limitatif, une préparation particulière comprend : la dissolution de 0,4 g de roténone purifiée à 99,2 % dans 15 g de benzoate de lauryle; addition de 0,15 g de nanosphères de polysiloxane, de 60 à 300 nm, dispersées dans 23 g d'éthanol; dilution de l'ensemble avec 61 g d'hexaméthyl disiloxane, solvant connu dans le commerce sous la dénomination de "DIMETHYLCONE", qui assure l'homogénéité de la composition. Les teneurs % pondérales sont :

| | |
|---|---|
| roténone pure | 0,40 % |
| sphérules de polysiloxane | 0,15 % |
| benzoate de lauryle | 15,00 % |
| éthanol | 23,00 % |
| hexaméthyl disiloxane | 61,45 % |
| | 100,00 % |

Bien entendu, dans des compositions renfermant de l'éthanol ou un autre alcool, la proportion de celui-ci ne dépasse pas la teneur qui provoquerait la précipitation de la roténone.

Un autre mode opératoire consiste à remplacer la suspension B de sphérules par une poudre de celles-ci, la préparation de sphérules, même extrêmement petites, à l'état pulvérulent étant connue en soi.

Il est également possible de produire la composition selon l'invention en dispersant, dans la solution roténonique A, un monomère polymérisable, et à le soumettre à la polymérisation à l'état dispersé dans cette solution. Ce type de polymérisation est décrit dans la publication FR-2 649 888 (Exemple 2).

L'invention peut se pratiquer suivant une des formes d'exécution sus-indiquées, que le solvant de l'agent roténonique et de ses éventuels additifs soit miscible ou non avec le liquide qui tient en suspension les sphérules ; en l'absence de miscibilité, il convient d'ajouter un composé tensioactif approprié, permettant de former une émulsion stable.

Une variante des procédés décrits ci-dessus comprend une opération supplémentaire, à la fin, à savoir l'élimination des liquides présents. Cela peut être réalisé par une opération classique, telle que centrifugation, ultrafiltration, distillation - s'il y a lieu sous vide - précipitation par un non solvant, etc. On obtient ainsi l'insecticide voulu à l'état de poudre, fort pratique dans certains cas et avantageux au moins du fait qu'il dispense du choix d'un liquide bien supporté par les sujets traités.

Le produit peut aussi se présenter sous la forme d'une crème ou pâte convenant bien à certaines applications ; pour l'obtenir, le liquide de la composition peut être incomplètement éliminé, ou bien complètement, ce qui reste étant incorporé dans une crème, mousse ou pâte de qualité thérapeutique ou/et cosmétique.

L'invention est illustrée par les exemples non limitatifs qui suivent.

EXEMPLE 1

**Traitement de la pédiculose**.

Par dissolution dans de l'hexylène glycol on a préparé trois solutions roténoniques :

I - à 0,65 % d'extrait de roténone commercial, à 45 % de roténone;
II - à 0,3 % de roténone purifiée, titrant 99,3 % de ce composé;
III - à 0,3 % de roténone purifiée (99,3 %), réduite à la manière décrite dans l'exemple 9.

D'autre part, on a constitué quatre lots de sujets infestés par des poux, tous au même degré d'infestation, tant en ce qui concerne le nombre de poux que pour la vitalité de lentes. Un premier lot de 10 sujets n'ayant pas été traités, a servi de témoin, les trois autres lots, de 30 sujets chaque, ont subi des traitements respectivement avec les trois solutions I, II et III.

**EP 0 588 857 B1**

Le tableau 1 des résultats, ci-après, relate l'évolution pendant 4 jours (J-0 à J-4); J-0 c'est-à-dire le jour zéro, est celui de l'examen des sujets, de la sélection, du dénombrement, puis de la première application de l'insecticide.

Au jour J-1 : ont eu lieu un dénombrement et la deuxième application.

Au jour J-2 : un dénombrement et la troisième application.

Au jour J-3 : un dénombrement seulement.

Au jour J-4 : dénombrement des poux survivants.

Les nombres d'insectes vivants, trouvés, sont indiqués dans les colonnes verticales.

TABLEAU 1

| Composition | J-0 | J-1 | J-2 | J-3 | J-4 |
|-------------|-----|-----|-----|-----|-----|
| I | 10 | 4 | 2 | 3 | 6 |
| II | 10 | 2 | 3 | 2 | 3 |
| III | 10 | 2 | 1 | 2 | 0 |

On retrouve le comportement habituel avec la composition classique I, dont 3 applications n'empêchent pas les insectes de resurgir le quatrième jour. Ce phénomène est fortement atténué par la roténone pure, composition II, et semble supprimé par la troisième application (J-3) lorsqu'on utilise la roténone réduite (III). L'emploi de la roténone pure et de son produit de réduction apportent donc un progrès très marqué par rapport à celui de l'extrait roténonique commercial, habituel.

## EXEMPLE 2

Dans les conditions décrites à l'exemple 1, on a essayé, comparativement avec les compositions II et III des suspensions de sphérules de polysiloxane de diamètre moyen d'environ 100 nm :

| IV et | V |
|-------|---|
| 0,29 % roténone pure | 0,29 % roténone réduite |
| 0,50 % nanosphères | 0,50 % nanosphères |
| 99,21 % hexaméthylèneglycol | 99,21 % hexaméthylèneglycol |

Le tableau 2 réunit les résultats de ces essais.

TABLEAU 2

| Composition | J-0 | J-1 | J-2 | J-3 | J-4 |
|-------------|-----|-----|-----|-----|-----|
| II -- roténone pure | 10 | 3 | 2 | 2 | 4 |
| IV -- roténone pure + nanosphères | 10 | 3 | 1 | 0 | 0 |
| III -- roténone pure, réduite | 10 | 1 | 3 | 1 | 0 |
| V -- roténone pure, réduite + nanosphères | 10 | 2 | 0 | 0 | 0 |

On constate qu'en présence de sphérules de polymère la roténone pure et son produit de réduction (II et III) donnent des résultats encore plus remarquables qu'avec du solvant seul. Le tableau 2 montre bien qu'à partir du deuxième jour, la destruction des insectes est pratiquement complète; en outre, 0 au quatrième jour signifie que les lentes n'ont pas pu éclore, ce qui représente un avantage insigne.

5

EXEMPLE 3

A chacun des jours J-0, J-1, J-2, J-4 et J-8, des poux adultes, d'âges comparables, ayant été en contact avec des cheveux traités par les compositions suivant l'invention, furent répartis dans des boîtes. On a fait de même avec des poux prélevés sur des cheveux témoins, non traités. La mort ou la survie de ces insectes était constatée après 12 et après 24 heures.

Le tableau 3 donne les nombres de poux vivants, au départ et après 12 et 24 heures.

TABLEAU 3

| Composition III, soit solution de 0,29 % de roténone réduite. | | | |
|---|---|---|---|
| | Au départ | Après 12 h | Après 24 h |
| J-0 | 40 | 32 | 4 |
| J-1 | 17 | 7 | 0 |
| J-2 | 16 | 13 | 1 |
| J-4 | 15 | 8 | 2 |
| J-8 | 13 | 11 | 0 |
| Composition V (III + nanosphères) | | | |
| J-0 | 14 | 4 | 0 |
| J-1 | 64 | 5 | 0 |
| J-2 | 16 | 8 | 0 |
| J-4 | 13 | 11 | 0 |
| J-8 | 15 | 12 | 0 |

Il est frappant de voir qu'avec la composition V, aucun survivant ne reste; quel que soit le nombre d'applications; notamment, puisque la ligne J-0 correspond à la première application, comme exposé dans l'exemple 1, on voit que cette unique application suffit pour que tous les poux soient tués au bout de 24 heures, s'il sont touchés par la composition V. C'est un avantage de valeur, puisqu'avec d'autres insecticides il faut toujours plusieurs applications. Il y a donc rémanence du principe actif.

EXEMPLE 4

*Lotion capillaire*

| Roténone purifiée, à 99 % | 0,35 g |
|---|---|
| Nanosphère de polystyrène-méthacrylate de méthyle (60/40) | 0,60 g |
| Essence de lavande | 1,00 g |
| Hexylène glycol | 98,05 g |

EP 0 588 857 B1

EXEMPLE 5

*Une autre lotion capillaire*

| Roténone à 99 % réduite | 0,3 g |
|---|---|
| Essence de lavande | 1,0 g |
| Thymol | 0,5 g |
| Butylène glycol | 98,2 g |

EXEMPLES 6 A 8

*Lotions capillaires*

| | 6 | 7 | 8 |
|---|---|---|---|
| Roténone pure, à 99,5 % | 0,3 | - | 0,3 g |
| Roténone pure réduite | - | 0,3 | - |
| Essence de lavande | 0,5 | 0,5 | 0,5 |
| Thymol | 0,2 | 0,2 | 0,2 |
| Sphérules 60-300 nm de polysiloxane | - | 0,6 | 0,5 |
| Benzoate de lauryle | 40 | 25,4 | 40 |
| Poly-diméthyl cyclosiloxane | 59 | 74 | 58,5 |

EXEMPLE 9

*Préparation de la roténone réduite*

A partir d'un extrait naturel de roténone on prépare d'abord une roténone pure à 99,2 %, 10 g de ce produit pur, soit 0,025 mole de roténone, sont mis en suspension dans 50 ml de méthanol, et la suspension est maintenue à 0°C. Des pastilles de borohydrure de sodium $NaBH_4$ sont alors introduites lentement dans la suspension.

Quand le dégagement d'hydrogène a cessé, on filtre; aux solides séparés, on ajoute 200 ml d'éther diéthylique, puis 200 ml d'eau, en agitant vigoureusement.

Le milieu liquide est ensuite additionné d'environ 40 ml d'HCl N, jusqu'à pH 6,5 à 7, après quoi on laisse décanter et l'on prélève la phase éthérée. On procède alors à trois lavages successifs de cette phase, par agitation, chaque fois, avec 250 ml d'eau, décantation et séparation de la phase éthérée. Celle-ci est finalement séchée sur du sulfate de sodium, filtrée et évaporée, ce qui laisse 10 g de roténone réduite, pure.

**Nota** : Les sujets de l'expérimentation étaient des enfants de 6 à 10 ans.

**Revendications**

1. Composition insecticide comprenant de la roténone et/ou de la déhydroroténone en tant qu'agent actif, caractérisée en ce que la roténone et/ou déhydroroténone est pure à au moins 90%.

2. Composition selon la revendication 1, contenant de 0,05% à 10% en poids de roténone et/ou déhydroroténone.

3. Composition selon la revendication 2, contenant de 0,1 à 5% en poids de roténone et/ou déhydroroténone.

4. Composition selon la revendication 1, 2 ou 3, dans laquelle la roténone et/ou déhydroroténone est pure à au moins 99%.

5. Composition selon l'une des revendications 1 à 4, dans laquelle ledit agent actif est fixé sur des sphérules en polymère de petites dimensions.

6. Composition selon la revendication 5, caractérisée en ce qu'elle est pulvérulente.

7. Composition selon la revendication 5, caractérisée en ce qu'elle est constituée par une pâte, une crème ou une mousse.

8. Composition selon la revendication 5, caractérisée en ce qu'elle est sous la forme d'un liquide contenant en suspension lesdites sphérules qui ont fixé l'agent actif.

9. Composition selon la revendication 8, caractérisée en ce qu'elle est à l'état d'émulsion.

10. Composition selon la revendication 8 ou 9, dans laquelle lesdites sphérules sont en suspension dans un liquide comprenant un solvant dudit agent actif.

11. Composition selon la revendication 10, dans laquelle ledit solvant est choisi dans le groupe consistant en esters d'acide phtalique, benzoïque, benzylique, phosphoreux, phosphorique ou méllitique, alkylène-glycol à chaîne carbonée de $C_2$ à $C_{18}$, et polyalkyl siloxane linéaire ou cyclique.

12. Composition selon l'une des revendications 5 à 11, dans laquelle le polymère constituant lesdites sphérules est choisi parmi les polysaccharides, polyamides, polyalkylènes, polyaryl-alkylènes, polyalkylidènes et polysiloxanes.

13. Application de la composition selon l'une quelconque des revendications 5 à 12 à la destruction des parasites du corps humain ou animal dans laquelle ladite composition contient en poids de 0,1 à 3% desdites sphérules.

14. Médicament conforme à l'application selon la revendication 13, dans lequel lesdites sphérules sont en émulsion dans un liquide contenant essentiellement du benzoate de lauryle, de l'éthanol et de l'héxaméthyl-disiloxane.

15. Procédé de préparation d'une composition selon l'une des revendications 5 à 12, caractérisé en ce que lesdites sphérules sont ajoutées à l'état de suspension dans un liquide ne les dissolvant pas.

16. Procédé de préparation d'une composition selon la revendication 15, caractérisé en ce que, si le liquide de la suspension n'est pas miscible avec la solution de l'agent actif, on ajoute un composé tensioactif, de façon à créer une émulsion stable, ou bien un solvant tiers.

17. Procédé de préparation d'une composition selon l'une des revendications 5 à 12, dans lequel le polymère constituant lesdites sphérules est obtenu en polymérisant un monomère polymérisable dispersé préalablement dans un liquide contenant ledit agent actif.

18. Procédé selon l'une des revendications 15, 16, 17, dans lequel on procède in fine à l'élimination des liquides présents dans la composition par centrifugation, ultra filtration, distillation, ou précipitation.

**Claims**

1. Insecticide composition comprising rotenone and/or dihydrorotenone as active agent, characterised in that the rotenone or dihydrorotenone is at least 90% pure.

2. Composition according to claim 1, containing from 0.05% to 10% by weight of rotenone and/or dihydrorotenone.

3. Composition according to claim 2, containing from 0.1% to 5% by weight of rotenone and/or dihydrorotenone.

4. Composition according to claim 1, 2 or 3, wherein the rotenone and/or dihydrorotenone is at least 99% pure.

5. Composition according to any one of claims 1 to 4, wherein said active agent is bound to polymer spherules of small dimensions.

6. Composition according to claim 5, characterised in that it is pulverulent.

7. Composition according to claim 5, characterised in that it is in the form of a paste, a cream or a foam.

8. Composition according to claim 5, characterised in that it is in the form of a liquid containing a suspension of said spherules which have the active agent bound thereto.

9. Composition according to claim 8, characterised in that it is an emulsion.

10. Composition according to claim 8 or 9, wherein said spherules are in suspension in a liquid comprising a solvent of said active agent.

11. Composition according to claim 10, wherein said solvent is selected from the group consisting in an ester of phthalic, benzoic, benzylic, phosphorous, phosphoric, or mellitic acid, a $C_2$ to $C_{18}$ alkylene-glycol, and linear or cyclic polyalkyl-siloxane.

12. Composition according to one of the claims 5 to 11, wherein the polymer forming said spherules is selected from polysaccharides, polyamides, polyalkylenes, polyarylalkylenes, polyalkylidenes and polysiloxanes.

13. Application of the composition according to any one of the claims 5 to 12 to obtain a drug for destroying parasites on the human or animal body, said drug containing from 0.1 to 3% of said spherules by weight.

14. Drug complying with the application according to claim 13, wherein said spherules are in emulsion in a liquid essentially containing lauryl benzoate, ethanol and hexamethyl-disiloxane.

15. Process of preparation of a composition according to any one of the claims 5 to 12, characterised in that said spherules are added in the form of a suspension in a liquid not dissolving them.

16. Process of preparation according to claim 15, characterised in that, if the liquid of the suspension is not miscible with the solution of the active agent, a surface-active agent is added in order to create a steady emulsion, or a third solvent.

17. Process of preparation of a composition according to any one of the claims 5 to 12, wherein the polymer forming said spherules is obtained by polymerising a polymerisable monomer which is previously dispersed in a liquid containing said active agent.

18. Process according to any one of the claims 15, 16, 17, wherein the liquids which are in the composition are removed in fine by centrifugation, ultra filtration, distillation or precipitation.

## Patentansprüche

1. Insektizidzusammensetzung, die als Wirkstoff Rotenon und/oder Dehydrorotenon enthält, dadurch gekennzeichnet, daß das Rotenon und/oder Dehydrorotenon zu mindestens 90 % rein ist.

2. Zusammensetzung nach Anspruch 1, die 0,05 bis 10 Gew.-% Rotenon und/oder Dehydrorotenon enthält.

3. Zusammensetzung nach Anspruch 2, die 0,1 bis 5 Gew.-% Rotenon und/oder Dehydrorotenon enthält.

4. Zusammensetzung nach Anspruch 1, 2 oder 3, in der das Rotenon und/oder Dehydrorotenon zu mindestens 99 % rein ist.

5. Zusammensetzung nach einem der Ansprüche 1 bis 4, in der der Wirkstoff an Polymerkügelchen kleiner Abmessungen fixiert ist.

6. Zusammensetzung nach Anspruch 5, dadurch gekennzeichnet, daß sie pulverförmig ist.

7. Zusammensetzung nach Anspruch 5, dadurch gekennzeichnet, daß sie aus einer Paste, einer Creme oder einem Schaum besteht.

8. Zusammensetzung nach Anspruch 5, dadurch gekennzeichnet, daß sie in Form einer Flüssigkeit vorliegt, die die Kügelchen, an die der Wirkstoff fixiert ist, in Suspension enthält.

9. Zusammensetzung nach Anspruch 8, dadurch gekennzeichnet, daß sie im Zustand von Emulsion ist.

10. Zusammensetzung nach Anspruch 8 oder 9, in der die Kügelchen in einer ein Lösungsmittel des Wirkstoffs enthaltenden Flüssigkeit in Suspension sind.

11. Zusammensetzung nach Anspruch 10, in der das Lösungsmittel aus der Gruppe ausgewählt ist, die aus Estern der Phthalsäure, Benzoesäure, Benzylsäure, phosphorigen Säure, Phosphorsäure oder Mellithsäure, Alkylen-Glycol mit Kohlenstoffkette mit 2 bis 18 Kohlenstoffatomen und linearem oder zyklischem Polyalkylsiloxan besteht.

12. Zusammensetzung nach einem der Ansprüche 5 bis 11, in der das die Kügelchen bildende Polymer aus den Polysacchariden, Polyamiden, Polyalkylenen, Polyarylalkylenen, Polyalkylidenen und Polysiloxanen ausgewählt ist.

13. Anwendung der Zusammensetzung nach einem der Ansprüche 5 bis 12 auf die Vernichtung von Parasiten des menschlichen oder tierischen Körpers, in der die Zusammensetzung 0,1 bis 3 Gew.-% Kügelchen enthält.

14. Arzneimittel gemäß der Anwendung nach Anspruch 13, in dem die Kügelchen in einer Flüssigkeit in Emulsion sind, die im wesentlichen Laurylbenzoat, Ethanol und Hexamethyl-disiloxan enthält.

15. Verfahren zur Herstellung einer Zusammensetzung nach einem der Ansprüche 5 bis 12, dadurch gekennzeichnet, daß die Kügelchen im Zustand der Suspension in einer Flüssigkeit, die sie nicht löst, beigegeben werden.

16. Verfahren zur Herstellung einer Zusammensetzung nach Anspruch 15, dadurch gekennzeichnet, daß, wenn die Flüssigkeit der Suspension nicht mit der Lösung des Wirkstoffs mischbar ist, man eine Tensidzusammensetzung beigibt, so daß eine stabile Emulsion geschaffen wird, oder ein Drittlösungsmittel .

17. Verfahren zur Herstellung einer Zusammensetzung nach einem der Ansprüche 5 bis 12, in dem das die Kügelchen bildende Polymer erhalten wird, indem man ein polymerisierbares Monomer polymerisiert, das zuvor in einer den Wirkstoff enthaltenden Flüssigkeit dispergiert wurde.

18. Verfahren nach einem der Ansprüche 15, 16, 17, in dem man zum Schluß die in der Zusammensetzung vorhandenen Flüssigkeiten durch Zentrifugieren, Filtrieren, Destillieren oder Ausfällen entfernt.